# EUROPÄISCHE PATENTANMELDUNG

(11) **EP 4 792 863 A1**
(43) Veröffentlichungstag der Anmeldung: **19.08.2026**
(21) Anmeldenummer: 26158130.0
(22) Anmeldetag: 12.02.2026
(51) Int. Cl.: A61M 16/00, A61M 16/10, A61M 16/16

(54) **STEUERN EINER FEUCHTIGKEIT UND EINER TEMPERATUR EINES ATEMLUFTSTROMS**

(30) Priorität: 18.02.2025 DE 102025106103
(71) Anmelder: Löwenstein Medical Technology S.A., 2557 Luxembourg (LU)
(72) Erfinder: Sartor, Jürgen, 76185 Karlsruhe (DE)
(74) Vertreter: Löwenstein Medical IP

(57) **Zusammenfassung**

Ein Verfahren zum Steuern einer Feuchtigkeit und einer Temperatur eines Atemluftstroms (17) umfasst: Steuern der Feuchtigkeit in mehreren aufeinanderfolgenden Zeitschritten in einem Feuchtigkeitsregelkreis, wobei in jedem Zeitschritt ein Feuchtigkeitssollwert (35) und ein Feuchtigkeitsistwert (37) empfangen werden, eine Feuchtigkeitsabweichung durch Vergleich des Feuchtigkeitssollwerts mit dem Feuchtigkeitsistwert bestimmt wird und mindestens ein Feuchtigkeitssteuerparameter (39) zur Regelung der Leistung einer Befeuchtungseinrichtung (11) angepasst wird, um die Feuchtigkeitsabweichung zu verringern; Steuern der Temperatur in mehreren aufeinanderfolgenden Zeitschritten in einem Temperaturregelkreis, wobei in jedem Zeitschritt ein Temperatursollwert (41) und ein Temperaturistwert (43) empfangen werden, eine Temperaturabweichung durch Vergleich des Temperatursollwerts mit dem Temperaturistwert bestimmt wird und mindestens ein Temperatursteuerparameter (45) zur Regelung der Leistung einer Temperiereinrichtung (21) angepasst wird, um die Temperaturabweichung zu verringern. Der Feuchtigkeitssteuerparameter kann zusätzlich die Temperaturabweichung und/oder den Temperatursteuerparameter berücksichtigen; analog kann der Temperatursteuerparameter zusätzlich die Feuchtigkeitsabweichung und/oder den Feuchtigkeitssteuerparameter berücksichtigen.

## Beschreibung

### Technisches Gebiet

Die Erfindung betrifft ein Verfahren zum Steuern einer Feuchtigkeit und einer Temperatur eines Atemluftstroms mittels einer Steuereinheit eines Atemluftbefeuchtungssystems. Des Weiteren betrifft die Erfindung eine Steuereinheit, ein Computerprogramm und ein computerlesbares Medium zum Ausführen eines solchen Verfahrens und ein Atemluftbefeuchtungssystem mit einer solchen Steuereinheit.

### Stand der Technik

Beatmungspatienten können manchmal unter zu trockenen Atemwegen leiden. In solchen Fällen kann die einzuatmende Atemluft mithilfe eines Atemluftbefeuchters zusätzlich befeuchtet werden. Somit kann eine Austrocknung der Schleimhäute vermieden oder zumindest abgemildert werden. Um die derart befeuchtete Atemluft zusätzlich temperieren zu können, kann eine Atemluftleitung, die einen Atemluftausgang des Atemluftbefeuchters mit einer geeigneten Patientenschnittstelle (beispielsweise einer Maske oder einer Nasenkanüle) verbindet, mittels einer zusätzlichen elektrischen Heizeinrichtung (beispielsweise einer Schlauchheizung) beheizbar sein. Die parallele Steuerung der Feuchtigkeit und der Temperatur des durch die Atemluftleitung geleiteten Atemluftstroms kann abhängig von den jeweiligen Umgebungsbedingungen mit gewissen Schwierigkeiten verbunden sein.

### Offenbarung der Erfindung

Eine Aufgabe der Erfindung kann darin gesehen werden, ein Verfahren zu schaffen, das auch bei stärker schwankenden Umgebungsbedingungen ein zuverlässiges Steuern der Feuchtigkeit und der Temperatur des Atemluftstroms ermöglicht. Eine weitere Aufgabe der Erfindung kann darin gesehen werden, eine Steuereinheit, ein Computerprogramm und ein computerlesbares Medium zum Ausführen eines solchen Verfahrens sowie ein entsprechendes Atemluftbefeuchtungssystem bereitzustellen.

Diese Aufgaben werden durch die Gegenstände der unabhängigen Ansprüche gelöst. Vorteilhafte Ausführungsformen der Erfindung sind in den abhängigen Ansprüchen, der nachfolgenden Beschreibung und den beigefügten Figuren dargelegt.

Ein erster Aspekt der Erfindung betrifft ein Verfahren zum Steuern einer Feuchtigkeit und einer Temperatur eines Atemluftstroms mittels einer Steuereinheit eines Atemluftbefeuchtungssystems.

Das Atemluftbefeuchtungssystem umfasst zusätzlich zur Steuereinheit einen Atemluftbefeuchter mit einem Atemlufteingang, einem Atemluftausgang, einem Flüssigkeitsbehälter und einer elektrisch steuerbaren Befeuchtungseinrichtung, die ausgebildet ist, um eine wasserhaltige Flüssigkeit aus dem Flüssigkeitsbehälter in Wasserdampf und/oder Wassertröpfchen umzuwandeln. Der Atemluftbefeuchter ist ausgebildet, um Atemluft, die am Atemlufteingang zugeführt wird, durch Vermischen mit dem Wasserdampf und/oder den Wassertröpfchen zu befeuchten und im derart befeuchteten Zustand als den Atemluftstrom am Atemluftausgang bereitzustellen. Des Weiteren umfasst das Atemluftbefeuchtungssystem eine elektrisch steuerbare Temperiereinrichtung, die ausgebildet ist, um den Atemluftstrom zusätzlich zu temperieren, einen Feuchtigkeitssensor zum Erfassen der Feuchtigkeit des Atemluftstroms und einen Temperatursensor zum Erfassen der Temperatur des Atemluftstroms.

Das Verfahren umfasst: Steuern der Feuchtigkeit des Atemluftstroms in mehreren aufeinanderfolgenden Zeitschritten in einem Feuchtigkeitsregelkreis, wobei in jedem Zeitschritt des Feuchtigkeitsregelkreises ein Feuchtigkeitssollwert und ein Feuchtigkeitsistwert, der eine mittels des Feuchtigkeitssensors erfasste Feuchtigkeit des Atemluftstroms anzeigt, empfangen werden, eine Feuchtigkeitsabweichung durch Vergleichen des Feuchtigkeitssollwerts mit dem Feuchtigkeitsistwert bestimmt wird und mindestens ein Feuchtigkeitssteuerparameter zum Steuern einer Leistung, mit der die Befeuchtungseinrichtung die Flüssigkeit in den Wasserdampf und/oder die Wassertröpfchen umwandelt, angepasst wird, um die Feuchtigkeitsabweichung zu verringern; Steuern der Temperatur des Atemluftstroms in mehreren aufeinanderfolgenden Zeitschritten in einem Temperaturregelkreis (beispielsweise parallel zum Steuern der Feuchtigkeit im Feuchtigkeitsregelkreis), wobei in jedem Zeitschritt des Temperaturregelkreises ein Temperatursollwert und ein Temperaturistwert, der eine mittels des Temperatursensors erfasste Temperatur des Atemluftstroms anzeigt, empfangen werden, eine Temperaturabweichung durch Vergleichen des Temperatursollwerts mit dem Temperaturistwert bestimmt wird und mindestens ein Temperatursteuerparameter zum Steuern einer Leistung, mit der die Temperiereinrichtung den Atemluftstrom zusätzlich temperiert, angepasst wird, um die Temperaturabweichung zu verringern.

Dabei wird der mindestens eine Feuchtigkeitssteuerparameter (beispielsweise in mindestens einem oder jedem der Zeitschritte des Feuchtigkeitsregelkreises) unter zusätzlicher Berücksichtigung der Temperaturabweichung und/oder des mindestens einen Temperatursteuerparameters aus mindestens einem der Zeitschritte des Temperaturregelkreises angepasst.

Zusätzlich oder alternativ wird der mindestens eine Temperatursteuerparameter (beispielsweise in mindestens einem oder jedem der Zeitschritte des Temperaturregelkreises) unter zusätzlicher Berücksichtigung der Feuchtigkeitsabweichung und/oder des mindestens einen Feuchtigkeitssteuerparameters aus mindestens einem der Zeitschritte des Feuchtigkeitsregelkreises angepasst.

Anders ausgedrückt können der Feuchtigkeitsregelkreis und der Temperaturregelkreis miteinander gekoppelt sein, um Wechselwirkungen zwischen den beiden Regelkreisen gezielt zu beeinflussen, sodass insgesamt eine hohe Regelgüte erreicht wird. Dies kann sich vorteilhaft auf die Zuverlässigkeit und/oder Genauigkeit beim (parallelen) Steuern der Feuchtigkeit und der Temperatur des Atemluftstroms, insbesondere bei stärker schwankenden Umgebungsbedingungen wie beispielsweise der Umgebungstemperatur, der Umgebungsfeuchtigkeit oder des Umgebungsdrucks, auswirken.

Ein weiterer Vorteil ist, dass dazu nicht zwingend weitere Informationen erforderlich sind, beispielsweise über einen Volumenstrom des Atemluftstroms, die jeweilige Umgebungstemperatur oder den jeweiligen Umgebungsdruck.

Da zwei verschiedene Sensortypen zum Einsatz kommen, kann zudem eine Berücksichtigung oder ein Ausgleich von Messfehlern, wie sie typischerweise beim Einsatz mehrerer Sensoren vom gleichen Typ auftreten, entfallen. Dies kann die Implementierung der Steuerung insgesamt vereinfachen.

Eine derartige Steuerung erfordert zudem keine komplexen mathematischen Modelle in Bezug auf die physikalischen Eigenschaften des Atemluftbefeuchters und/oder einer daran angeschlossenen Atemluftleitung (beispielsweise eines beheizbaren Beatmungsschlauchs). Vielmehr funktioniert eine derartige Steuerung unabhängig (oder zumindest weitgehend unabhängig) von solchen Modellen, solange die Leistung der Befeuchtungs- und/oder Temperiereinrichtung ausreichend ist.

Das Verfahren kann beispielsweise computerimplementiert sein.

Unter "Feuchtigkeit" kann beispielsweise eine absolute Luftfeuchtigkeit und/oder eine relative Luftfeuchtigkeit bezogen auf die jeweilige Umgebungstemperatur verstanden werden.

Unter "Atemluft" kann ein einzelnes Atemgas oder ein Gemisch aus mehreren Atemgasen verstanden werden. Unter "Atemgas" kann beispielsweise Kohlendioxid, Sauerstoff, Stickstoff, Wasserdampf oder Narkosegas verstanden werden.

Die Feuchtigkeitsabweichung kann beispielsweise durch Bilden einer Differenz zwischen dem Feuchtigkeitssollwert und dem Feuchtigkeitsistwert bestimmt werden. In entsprechender Weise kann die Temperaturabweichung beispielsweise durch Bilden einer Differenz zwischen dem Temperatursollwert und dem Temperaturistwert bestimmt werden.

Unter "Istwert" wie in "Feuchtigkeitsistwert" oder "Temperaturistwert" kann ein mittels des jeweiligen Sensors bestimmter Messwert und/oder Schätzwert (beispielsweise ein Mittelwert) verstanden werden.

Unter "Steuerparameter" wie in "Feuchtigkeitssteuerparameter" oder "Temperatursteuerparameter" kann beispielsweise einer der folgenden Parameter verstanden werden: ein elektrischer Strom, der zwischen den elektrischen Anschlüssen der Befeuchtungs- bzw. Temperiereinrichtung fließt; eine elektrische Spannung, die an den elektrischen Anschlüssen der Befeuchtungs- bzw. Temperiereinrichtung anliegt; eine Frequenz, mit der die Befeuchtungs- bzw. Temperiereinrichtung abwechselnd ein- und ausgeschaltet wird; ein zeitliches Verhältnis zwischen Einschaltphasen, in denen die Befeuchtungs- bzw. Temperiereinrichtung eingeschaltet ist, und Ausschaltphasen, in denen die Befeuchtungs- bzw. Temperiereinrichtung ausgeschaltet ist. Beispielsweise kann es sich bei der Frequenz um eine Taktfrequenz und/oder bei dem zeitlichen Verhältnis um einen Tastgrad im Kontext einer Pulsweitenmodulation handeln.

Beispielsweise kann die Leistung der Befeuchtungseinrichtung durch entsprechendes Anpassen des mindestens einen Feuchtigkeitssteuerparameters erhöht werden, wenn der Feuchtigkeitsistwert im Vergleich zum Feuchtigkeitssollwert eine zu geringe Feuchtigkeit anzeigt, und umgekehrt. Dabei kann das Anpassen des mindestens einen Feuchtigkeitssteuerparameters (in einer oder beiden Richtungen) unter zusätzlicher Berücksichtigung einer aktuellen Temperatur des Atemluftstroms (beispielsweise des jeweiligen Temperaturistwerts), einer aktuellen Temperaturabweichung und/oder einer aktuellen Leistung der Temperiereinrichtung (beispielsweise eines aktuellen Werts des jeweiligen Temperatursteuerparameters) erfolgen, um eine bestmögliche Annäherung der beiden Istwerte an ihren jeweiligen Sollwert zu erreichen.

Zusätzlich oder alternativ kann beispielsweise die Leistung der Temperiereinrichtung durch entsprechendes Anpassen des mindestens einen Temperatursteuerparameters erhöht werden, wenn der Temperaturistwert im Vergleich zum Temperatursollwert eine zu geringe Temperatur anzeigt, und umgekehrt. Dabei kann das Anpassen des mindestens einen Temperatursteuerparameters (in einer oder beiden Richtungen) unter zusätzlicher Berücksichtigung einer aktuellen Feuchtigkeit des Atemluftstroms (beispielsweise des jeweiligen Feuchtigkeitsistwerts), einer aktuellen Feuchtigkeitsabweichung und/oder einer aktuellen Leistung der Befeuchtungseinrichtung (beispielsweise eines aktuellen Werts des jeweiligen Feuchtigkeitssteuerparameters) erfolgen, um eine bestmögliche Annäherung der beiden Istwerte an ihren jeweiligen Sollwert zu erreichen.

Prinzipiell kann das Anpassen der vorgenannten Steuerparameter so erfolgen, dass sich die Feuchtigkeit und die Temperatur des Atemluftstroms jeweils einem gewünschten Betriebspunkt auf einer entsprechenden Kennlinie und/oder in einem entsprechenden Kennlinienfeld, beispielsweise einem *h*, *x*-Diagramm, annähern.

Beispielsweise kann das Steuern der Feuchtigkeit und/oder der Temperatur mithilfe einer PID-Regelung erfolgen. Je nach Anwendung sind aber auch andere Regelungstypen möglich.

Ein zweiter Aspekt der Erfindung betrifft eine Steuereinheit, die konfiguriert ist, um das vor- und nachstehend beschriebene Verfahren auszuführen.

Die Steuereinheit kann Mittel zur Datenverarbeitung umfassen. Die Mittel zur Datenverarbeitung können als Hard- und/oder Software implementiert sein und/oder einen Prozessor umfassen. Der Prozessor kann konfiguriert sein, um das (computerimplementierte) Verfahren auszuführen. Zusätzlich zum Prozessor kann die Steuereinheit mindestens eines der folgenden Mittel zur Datenverarbeitung umfassen: einen Speicher, ein Bussystem zur Datenkommunikation zwischen dem Speicher und dem Prozessor, eine Datenkommunikationsschnittstelle zur drahtlosen und/oder drahtgebundenen Datenkommunikation mit Peripheriegeräten. Alternativ kann die Steuereinheit ausschließlich als Hardware, beispielsweise in Form eines ASIC- oder FPGA-Bausteins, implementiert sein.

Es wird darauf hingewiesen, dass Merkmale des vor- und nachstehend beschriebenen Verfahrens auch Merkmale der Steuereinheit sein können (und umgekehrt).

Ein dritter Aspekt der Erfindung betrifft ein Atemluftbefeuchtungssystem. Das Atemluftbefeuchtungssystem umfasst einen Atemluftbefeuchter mit einem Atemlufteingang, einem Atemluftausgang, einem Flüssigkeitsbehälter und einer elektrisch steuerbaren Befeuchtungseinrichtung, die ausgebildet ist, um eine wasserhaltige Flüssigkeit aus dem Flüssigkeitsbehälter in Wasserdampf und/oder Wassertröpfchen umzuwandeln. Der Atemluftbefeuchter ist ausgebildet, um Atemluft, die am Atemlufteingang zugeführt wird, durch Vermischen mit dem Wasserdampf und/oder den Wassertröpfchen zu befeuchten und im derart befeuchteten Zustand als einen Atemluftstrom am Atemluftausgang bereitzustellen. Des Weiteren umfasst das Atemluftbefeuchtungssystem eine Atemluftleitung zum Anschließen an den Atemluftausgang, sodass der Atemluftstrom durch die Atemluftleitung geleitet wird, eine elektrisch steuerbare Temperiereinrichtung, die ausgebildet ist, um den Atemluftstrom zusätzlich zu temperieren, einen Feuchtigkeitssensor zum Erfassen einer Feuchtigkeit des Atemluftstroms, einen Temperatursensor zum Erfassen einer Temperatur des Atemluftstroms und eine Steuereinheit, wie sie vor- und nachstehend beschrieben wird.

Der Atemlufteingang kann beispielsweise an ein Beatmungsgerät zum invasiven und/oder nicht invasiven Beatmen eines Patienten (genauer an einen Druckausgang des Beatmungsgeräts) anschließbar sein, um eine Befeuchtung der vom Beatmungsgerät bereitgestellten Atemluft zu ermöglichen, bevor die Atemluft vom Patienten eingeatmet wird. Unter "Beatmungsgerät" kann auch ein Anästhesiegerät oder ein Hustenunterstützungsgerät zum Unterstützen eines Patienten beim Husten, auch Insufflator-Exsufflator genannt, verstanden werden.

Unter "Atemluftleitung" kann beispielsweise ein Schlauch, ein Rohr oder eine Kombination aus mindestens zwei dieser Beispiele verstanden werden. Die Atemluftleitung kann mit einem ersten ihrer Enden an den Atemluftausgang und/oder mit einem zweiten ihrer Enden an eine geeignete Patientenschnittstelle - beispielsweise eine Maske, eine Nasenkanüle und/oder einen Tubus - anschließbar sein. Dementsprechend kann das erste Ende auch als ein dem Atemluftausgang zugewandtes Ende der Atemluftleitung und/oder kann das zweite Ende auch als ein dem Atemluftausgang abgewandtes Ende der Atemluftleitung aufgefasst werden. Anders ausgedrückt kann der Atemluftausgang über die Atemluftleitung und eine geeignete Patientenschnittstelle an den Atemapparat eines Patienten anschließbar sein, sodass eine Beatmung des Patienten mit dem Atemluftstrom möglich ist.

Unter "Wassertröpfchen" können besonders kleine Wassertröpfchen eines Aerosols verstanden werden. Die Wassertröpfchen können beispielsweise eine durchschnittliche Partikelgröße von 50 µm oder weniger, von 10 µm oder weniger oder von 5 µm oder weniger haben. Hingegen kann es sich bei dem Wasserdampf um Wasser im gasförmigen Zustand handeln. Der Wasserdampf kann durch gezieltes (direktes und/oder indirektes) Erwärmen und entsprechendes Verdunsten der (wasserhaltigen) Flüssigkeit erzeugt werden.

Beispielsweise kann die Befeuchtungseinrichtung und/oder kann die Temperiereinrichtung eine elektrische Heizeinrichtung umfassen. Eine solche Heizeinrichtung kann beispielsweise ein Heizwiderstand in Form eines Heizstabs, einer Heizwendel, einer Heizplatte oder einer Kombination aus mindestens zwei dieser Beispiele sein.

Zusätzlich oder alternativ kann die Befeuchtungseinrichtung eine elektrische Zerstäubungseinrichtung zum Zerstäuben der Flüssigkeit, d. h. zum Umwandeln der Flüssigkeit in (besonders kleine) Wassertröpfchen, beispielsweise in ein Aerosol, umfassen. Eine solche Zerstäubungseinrichtung kann beispielsweise ein piezoelektrischer Ultraschallzerstäuber sein.

Die Temperiereinrichtung kann ausgebildet sein, um den Atemluftstrom direkt und/oder indirekt - beispielsweise durch entsprechendes Temperieren zumindest eines Abschnitts der Atemluftleitung - zusätzlich zu temperieren.

Die Temperiereinrichtung kann auch ausgebildet sein, um den Atemluftstrom und/oder zumindest einen Abschnitt der Atemluftleitung aktiv und/oder passiv zu kühlen. Dies hat den Vorteil, dass die beiden Sollwerte auch bei höheren Umgebungstemperaturen und/oder größeren Schwankungen der Umgebungstemperatur zuverlässig erreicht werden können.

Die Steuereinheit kann konfiguriert sein, um zusätzlich einen oder mehrere elektropneumatische Aktoren zum Bereitstellen des Atemluftstroms zu steuern, beispielsweise derart, dass sich die Feuchtigkeitsabweichung verringert und/oder sich die Temperaturabweichung verringert. Ein solcher elektropneumatischer Aktor kann beispielsweise ein Gebläse oder ein Ventil sein.

Es ist möglich, dass der Feuchtigkeitssensor und der Temperatursensor in einem gemeinsamen Sensorgehäuse angeordnet sind. Anders ausgedrückt können der Feuchtigkeitssensor und der Temperatursensor als verschiedene Module ein und desselben Sensorbausteins ausgeführt sein. Solche Sensorbausteine sind zum einen besonders günstig und zum anderen besonders einfach zu montieren, beispielsweise an und/oder in der Atemluftleitung.

Der Atemluftbefeuchter kann als ein eigenständiges Gerät und/oder als eine einzelne Komponente (von mehreren Komponenten) eines medizintechnischen Geräts, insbesondere eines Beatmungsgeräts, ausgeführt sein.

Weitere Aspekte der Erfindung betreffen ein Computerprogramm und ein computerlesbares Medium, auf dem das Computerprogramm gespeichert ist.

Das Computerprogramm umfasst Befehle, die die vor- und nachstehend beschriebene Steuereinheit, beispielsweise einen Prozessor der Steuereinheit, beim Ausführen des Computerprogramms durch die Steuereinheit veranlassen, das vor- und nachstehend beschriebene Verfahren auszuführen.

Das computerlesbare Medium kann ein flüchtiger oder nicht flüchtiger Datenspeicher sein. Beispielsweise kann das computerlesbare Medium eine Festplatte, ein USB-Speichergerät (USB = *universal serial bus*)*,* ein RAM (*random-access memory*)*,* ein ROM (*read-only memory*)*,* ein EPROM (*erasable programmable read-only memory*)*,* ein EEPROM (*electrically erasable programmable read-only memory*)*,* ein Flash-Speicher oder eine Kombination aus mindestens zwei dieser Beispiele sein. Das computerlesbare Medium kann auch ein Datenkommunikationsnetzwerk, das das Herunterladen von Programmcode ermöglicht (z. B. über das Internet), oder eine Cloud sein.

Es wird darauf hingewiesen, dass Merkmale des vor- und nachstehend beschriebenen Verfahrens auch Merkmale des Computerprogramms und/oder des computerlesbaren Mediums sein können (und umgekehrt).

Im Folgenden werden verschiedene Ausführungsformen der Erfindung beschrieben. Diese Ausführungsformen sind nicht als Beschränkung des Umfangs der Erfindung zu verstehen.

Gemäß einer Ausführungsform kann der mindestens eine Feuchtigkeitssteuerparameter (beispielsweise in mindestens einem oder jedem der Zeitschritte des Feuchtigkeitsregelkreises) unter zusätzlicher Berücksichtigung eines früheren Feuchtigkeitsistwerts und/oder einer früheren Feuchtigkeitsabweichung aus mindestens einem dem jeweiligen Zeitschritt vorangehenden früheren Zeitschritt des Feuchtigkeitsregelkreises angepasst werden. Anders ausgedrückt kann der mindestens eine Feuchtigkeitssteuerparameter (beispielsweise in mindestens einem oder jedem der Zeitschritte des Feuchtigkeitsregelkreises) unter zusätzlicher Berücksichtigung einer (beispielsweise durchschnittlichen und/oder geschätzten) zeitlichen Änderung der erfassten Feuchtigkeit und/oder einer (beispielsweise durchschnittlichen und/oder geschätzten) zeitlichen Änderung der Feuchtigkeitsabweichung angepasst werden. Dies kann die Genauigkeit beim Steuern der Feuchtigkeit, beispielsweise beim Bestimmen des D-Anteils einer PID-Regelung der Feuchtigkeit, weiter verbessern. Denkbar ist auch, dass ein Mittelwert aus der (aktuellen) Feuchtigkeitsabweichung und der früheren Feuchtigkeitsabweichung bestimmt wird. Der so bestimmte Mittelwert kann dann beispielsweise zum Bestimmen des I-Anteils einer PID-Regelung der Feuchtigkeit verwendet werden.

Zusätzlich oder alternativ kann der mindestens eine Temperatursteuerparameter (beispielsweise in mindestens einem oder jedem der Zeitschritte des Temperaturregelkreises) unter zusätzlicher Berücksichtigung eines früheren Temperaturistwerts und/oder einer früheren Temperaturabweichung aus mindestens einem dem jeweiligen Zeitschritt vorangehenden früheren Zeitschritt des Temperaturregelkreises angepasst werden. Anders ausgedrückt kann der mindestens eine Temperatursteuerparameter (beispielsweise in mindestens einem oder jedem der Zeitschritte des Temperaturregelkreises) unter zusätzlicher Berücksichtigung einer (beispielsweise durchschnittlichen und/oder geschätzten) zeitlichen Änderung der erfassten Temperatur und/oder einer (beispielsweise durchschnittlichen und/oder geschätzten) zeitlichen Änderung der Temperaturabweichung angepasst werden. Dies kann die Genauigkeit beim Steuern der Temperatur, beispielsweise beim Bestimmen des D-Anteils einer PID-Regelung der Temperatur, weiter verbessern. Denkbar ist auch, dass ein Mittelwert aus der (aktuellen) Temperaturabweichung und der früheren Temperaturabweichung bestimmt wird. Der so bestimmte Mittelwert kann dann beispielsweise zum Bestimmen des I-Anteils einer PID-Regelung der Temperatur verwendet werden.

Gemäß einer Ausführungsform kann der mindestens eine Feuchtigkeitssteuerparameter (beispielsweise in mindestens einem oder jedem der Zeitschritte des Feuchtigkeitsregelkreises) unter zusätzlicher Berücksichtigung der Temperaturabweichung aus mindestens einem der Zeitschritte des Temperaturregelkreises oder unter zusätzlicher Berücksichtigung sowohl der Temperaturabweichung als auch des mindestens einen Temperatursteuerparameters aus mindestens einem der Zeitschritte des Temperaturregelkreises angepasst werden, um - zusätzlich zur Feuchtigkeitsabweichung - die Temperaturabweichung zu verringern. Anders ausgedrückt kann die Leistung der Befeuchtungseinrichtung (beispielsweise deren Heizleistung) zusätzlich entsprechend verringert werden, wenn der Temperaturistwert im Vergleich zum Temperatursollwert eine zu hohe Temperatur anzeigt, und umgekehrt. Auf diese Weise kann eine zusätzliche Verringerung bzw. Erhöhung der Temperatur des Atemluftstroms bewirkt werden.

Zusätzlich oder alternativ kann der mindestens eine Temperatursteuerparameter (beispielsweise in mindestens einem oder jedem der Zeitschritte des Temperaturregelkreises) unter zusätzlicher Berücksichtigung der Feuchtigkeitsabweichung aus mindestens einem der Zeitschritte des Feuchtigkeitsregelkreises oder unter zusätzlicher Berücksichtigung sowohl der Feuchtigkeitsabweichung als auch des mindestens einen Feuchtigkeitssteuerparameters aus mindestens einem der Zeitschritte des Feuchtigkeitsregelkreises angepasst werden, um - zusätzlich zur Temperaturabweichung - die Feuchtigkeitsabweichung zu verringern. Anders ausgedrückt kann die Leistung der Temperiereinrichtung (beispielsweise deren Heizleistung) zusätzlich entsprechend verringert werden, wenn der Feuchtigkeitsistwert im Vergleich zum Feuchtigkeitssollwert eine zu geringe Feuchtigkeit anzeigt, und umgekehrt. Auf diese Weise kann eine zusätzliche Verringerung bzw. Erhöhung der Feuchtigkeit des Atemluftstroms bewirkt werden.

Gemäß einer Ausführungsform kann der mindestens eine Feuchtigkeitssteuerparameter nur dann angepasst werden, um zusätzlich die Temperaturabweichung zu verringern, wenn im jeweiligen Zeitschritt des Feuchtigkeitsregelkreises erkannt wird, dass die Temperiereinrichtung deaktiviert ist und/oder die Temperaturabweichung einen bestimmten Schwellenwert erreicht, beispielsweise über- oder unterschreitet. Dies kann die Effizienz des Verfahrens weiter verbessern.

Zusätzlich oder alternativ ist es möglich, dass der mindestens eine Temperatursteuerparameter nur dann angepasst wird, um zusätzlich die Feuchtigkeitsabweichung zu verringern, wenn im jeweiligen Zeitschritt des Temperaturregelkreises erkannt wird, dass die Befeuchtungseinrichtung deaktiviert ist und/oder die Feuchtigkeitsabweichung einen bestimmten Schwellenwert erreicht, beispielsweise über- oder unterschreitet. Dies kann die Effizienz des Verfahrens weiter verbessern.

Unter "Schwellenwert" kann vor- und nachstehend beispielsweise ein positiver oder negativer Zahlenwert verstanden werden. Anders ausgedrückt kann unter "Schwellenwert" ein Zahlenwert ungleich null verstanden werden. Durch Wählen eines geeigneten Schwellenwerts kann beispielsweise verhindert werden, dass der jeweilige Steuerparameter bereits bei einer sehr kleinen Feuchtigkeits- bzw. Temperaturabweichung angepasst wird. Dies kann die Regelgüte weiter verbessern.

Gemäß einer Ausführungsform kann in jedem Zeitschritt des Feuchtigkeitsregelkreises und/oder des Temperaturregelkreises zusätzlich ein Gewichtungswert, der eine Gewichtung des Feuchtigkeitsregelkreises gegenüber dem Temperaturregelkreis anzeigt, empfangen werden. In diesem Fall kann das Anpassen des mindestens einen Feuchtigkeitssteuerparameters und/oder des mindestens einen Temperatursteuerparameters im jeweiligen Zeitschritt unter zusätzlicher Berücksichtigung des Gewichtungswerts erfolgen. Auf diese Weise können auch deutlich voneinander abweichende Betriebspunkte, beispielsweise in einem h, x-Diagramm, eingestellt werden, ohne die Regelgüte des jeweils niedriger gewichteten Regelkreises übermäßig zu beeinträchtigen. Beispielsweise ist es möglich, dass das Anpassen des mindestens einen Feuchtigkeitssteuerparameters nur dann erfolgt, wenn der jeweilige Gewichtungswert einen bestimmten Schwellenwert erreicht und/oder dem Feuchtigkeitsregelkreis die gleiche oder eine höhere Gewichtung als dem Temperaturregelkreis zuordnet. Das Gleiche kann in entsprechender Weise für das Anpassen des mindestens einen Temperatursteuerparameters zutreffen.

Gemäß einer Ausführungsform kann das Anpassen des mindestens einen Feuchtigkeitssteuerparameters unter zusätzlicher Berücksichtigung des Gewichtungswerts umfassen: Bestimmen eines ersten Wertebereichs mit möglichen Werten für den mindestens einen Feuchtigkeitssteuerparameter unter Verwendung des Gewichtungswerts und einer ersten Zuordnungsvorschrift, die möglichen Gewichtungswerten jeweils einen von mehreren möglichen ersten Wertebereichen zuordnet; Anpassen des mindestens einen Feuchtigkeitssteuerparameters unter Verwendung des ersten Wertebereichs. Anders ausgedrückt können mögliche Einstellungen für den mindestens einen Feuchtigkeitssteuerparameter abhängig von der jeweiligen Gewichtung des Feuchtigkeitsregelkreises gegenüber dem Temperaturregelkreis gezielt begrenzt bzw. erweitert werden.

Unter "Wertebereich" wie in "erster Wertebereich" oder "zweiter Wertebereich" (siehe weiter unten) kann ein einzelner positiver oder negativer Wert (oder der Wert Null) oder eine Menge von mindestens zwei verschiedenen Werten verstanden werden.

Unter "Zuordnungsvorschrift" wie in "erster Zuordnungsvorschrift" oder "zweiter Zuordnungsvorschrift" (siehe weiter unten) kann beispielsweise eine mathematische Funktion und/oder eine Lookup-Tabelle verstanden werden. Eine solche Zuordnungsvorschrift kann beispielsweise in einem Speicher der Steuereinheit hinterlegt sein.

Gemäß einer Ausführungsform kann das Anpassen des mindestens einen Temperatursteuerparameters unter zusätzlicher Berücksichtigung des Gewichtungswerts umfassen: Bestimmen eines zweiten Wertebereichs mit möglichen Werten für den mindestens einen Temperatursteuerparameter unter Verwendung des Gewichtungswerts und einer zweiten Zuordnungsvorschrift, die möglichen Gewichtungswerten jeweils einen von mehreren möglichen zweiten Wertebereichen zuordnet; Anpassen des mindestens einen Temperatursteuerparameters unter Verwendung des zweiten Wertebereichs. Anders ausgedrückt können mögliche Einstellungen für den mindestens einen Temperatursteuerparameter abhängig von der jeweiligen Gewichtung des Temperaturregelkreises gegenüber dem Feuchtigkeitsregelkreis gezielt begrenzt bzw. erweitert werden.

Gemäß einer Ausführungsform kann der Feuchtigkeitssollwert eine relative Luftfeuchtigkeit von 100 % oder weniger, von 98 % oder weniger oder von 95 % oder weniger anzeigen. Mit Werten, die geringfügig unterhalb von 100 % liegen, können beispielsweise größere Ungenauigkeiten beim Steuern der Feuchtigkeit vermieden werden.

Gemäß einer Ausführungsform kann der Temperatursollwert eine Temperatur zwischen 30 °C und 40 °C, vorzugsweise zwischen 37 °C und 39 °C, anzeigen. Bei solchen Temperaturen wird der eingeatmete Atemluftstrom als besonders angenehm empfunden. Zudem kann ein derart temperierter Atemluftstrom eine Austrocknung der Schleimhäute besonders effektiv abmildern oder verhindern.

Gemäß einer Ausführungsform kann die Temperiereinrichtung ausgebildet sein, um den Atemluftstrom durch (beispielsweise aktives) Erwärmen und/oder (beispielsweise aktives) Kühlen zumindest eines Abschnitts der Atemluftleitung zusätzlich zu temperieren. Dies hat den Vorteil, dass die beiden Sollwerte auch bei höheren Umgebungstemperaturen und/oder größeren Schwankungen der Umgebungstemperatur zuverlässig erreicht werden können.

Gemäß einer Ausführungsform kann der Feuchtigkeitssensor ausgebildet sein, um die Feuchtigkeit des Atemluftstroms an mindestens einer Feuchtigkeitserfassungsstelle innerhalb eines dem Atemluftausgang abgewandten Endabschnitts der Atemluftleitung zu erfassen. Zusätzlich oder alternativ kann der Temperatursensor ausgebildet sein, um die Temperatur des Atemluftstroms an mindestens einer Temperaturerfassungsstelle innerhalb eines dem Atemluftausgang abgewandten Endabschnitts der Atemluftleitung zu erfassen. In beiden Fällen kann der Endabschnitt ein Längsabschnitt der Atemluftleitung sein, der sich ausgehend von einem dem Atemluftausgang abgewandten Ende der Atemluftleitung (in Längsrichtung der Atemluftleitung betrachtet) hin zu einem dem Atemluftausgang zugewandten, beispielsweise an den Atemluftausgang angeschlossenen Ende der Atemluftleitung über höchstens ein Drittel, höchstens ein Viertel, höchstens ein Zehntel oder höchstens ein Zwanzigstel einer (Gesamt-)Länge der Atemluftleitung erstreckt. Dies ermöglicht es, den Feuchtigkeitssensor und/oder den Temperatursensor möglichst nah am jeweiligen Patienten zu platzieren. Auf diese Weise kann im Vergleich zu einer Ausführungsform, bei der die Feuchtigkeit bzw. Temperatur des Atemluftstroms in einem sonstigen Längsabschnitt der Atemluftleitung außerhalb des Endabschnitts erfasst wird, erreicht werden, dass die tatsächliche Feuchtigkeit bzw. Temperatur des Atemluftstroms, kurz bevor dieser in eine an die Atemluftleitung angeschlossene Patientenschnittstelle eintritt, auch bei größeren Schwankungen der Umgebungsbedingungen mit hoher Genauigkeit einer gewünschten Feuchtigkeit bzw. Temperatur entspricht. Prinzipiell kann gesagt werden, dass der jeweilige Regelungsfehler umso kleiner ist, je näher der jeweilige Sensor am Patienten ist.

Zusätzlich oder alternativ kann die mindestens eine Feuchtigkeitserfassungsstelle und/oder kann die mindestens eine Temperaturerfassungsstelle in einem Hohlraum einer an die Atemluftleitung angeschlossenen Patientenschnittstelle, beispielsweise einer Maske, einer Nasenkanüle oder eines Tubus, liegen, wobei der Atemluftstrom im Betrieb des Atemluftbefeuchtungssystems aus der Atemluftleitung in den Hohlraum geleitet wird, sodass eine Erfassung der Feuchtigkeit und/oder der Temperatur des Atemluftstroms innerhalb des Hohlraums möglich ist.

### Kurze Beschreibung der Zeichnungen

Im Folgenden werden Ausführungsformen der Erfindung mit Bezug auf die beigefügten Zeichnungen beschrieben. Weder die Beschreibung noch die Zeichnungen sind als Beschränkung des Umfangs der Erfindung zu verstehen.
Fig. 1 zeigt ein Atemluftbefeuchtungssystem gemäß einer Ausführungsform der Erfindung.
Fig. 2 zeigt ein Ablaufdiagramm zur Veranschaulichung eines Verfahrens gemäß einer Ausführungsform der Erfindung.

Die Figuren sind rein schematisch und nicht maßstabsgetreu. Werden in verschiedenen Zeichnungen gleiche Bezugszeichen verwendet, so bezeichnen diese Bezugszeichen gleiche oder gleichwirkende Merkmale.

### Ausführungsformen der Erfindung

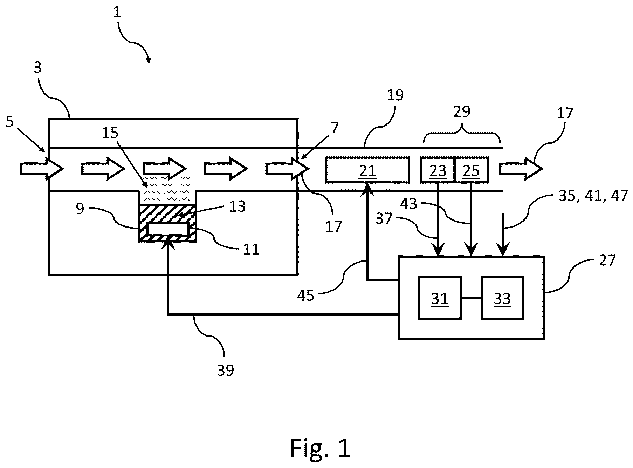

Fig. 1 zeigt ein Atemluftbefeuchtungssystem 1. Das Atemluftbefeuchtungssystem 1 umfasst einen Atemluftbefeuchter 3 mit einem Atemlufteingang 5, einem Atemluftausgang 7, einem Flüssigkeitsbehälter 9 und einer elektrisch steuerbaren Befeuchtungseinrichtung 11, hier in Form einer ersten elektrischen Heizeinrichtung. Die Befeuchtungseinrichtung 11 ist in diesem Beispiel ausgebildet, um eine wasserhaltige Flüssigkeit 13 aus dem Flüssigkeitsbehälter 9 in Wasserdampf 15 umzuwandeln. Der Atemluftbefeuchter 3 ist ausgebildet, um Atemluft, die am Atemlufteingang 5 zugeführt wird, durch Vermischen mit dem Wasserdampf 15 gezielt zu befeuchten und im derart befeuchteten Zustand als einen Atemluftstrom 17 am Atemluftausgang 7 bereitzustellen. Je nach Heizleistung der Befeuchtungseinrichtung 11 kann der Atemluftstrom 17, der am Atemluftausgang 7 austritt, mehr oder weniger feucht und/oder warm sein.

Des Weiteren umfasst das Atemluftbefeuchtungssystem 1 eine Atemluftleitung 19, die mit einem ihrer Enden an den Atemluftausgang 7 angeschlossen ist, sodass der Atemluftstrom 17 durch die Atemluftleitung 19 geleitet wird. Die Atemluftleitung 19 kann mit ihrem anderen, dem Atemluftausgang 7 abgewandten Ende an eine Patientenschnittstelle, beispielsweise eine Maske, eine Nasenkanüle oder einen Tubus, angeschlossen sein, sodass ein Patient den Atemluftstrom 17 über die Patientenschnittstelle einatmen kann.

Zudem umfasst das Atemluftbefeuchtungssystem 1 eine elektrisch steuerbare Temperiereinrichtung 21, die ausgebildet ist, um den Atemluftstrom 17 zusätzlich zu temperieren. Die Temperiereinrichtung 21 kann ausgebildet sein, um den Atemluftstrom 17 durch Erwärmen und/oder Kühlen zumindest eines Abschnitts der Atemluftleitung 19 zusätzlich zu temperieren. In diesem Beispiel ist die Temperiereinrichtung 21 als eine zweite elektrische Heizeinrichtung zum Beheizen zumindest eines Abschnitts der Atemluftleitung 19 ausgebildet.

Darüber hinaus umfasst das Atemluftbefeuchtungssystem 1 einen Feuchtigkeitssensor 23 zum Erfassen einer Feuchtigkeit des Atemluftstroms 17, einen Temperatursensor 25 zum Erfassen einer Temperatur des Atemluftstroms 17 und eine Steuereinheit 27 zum Steuern der Befeuchtungseinrichtung 11 und der Temperiereinrichtung 21 unter Verwendung von Sensordaten der beiden Sensoren 23, 25.

In diesem Beispiel ist der Feuchtigkeitssensor 23 ausgebildet, um die Feuchtigkeit des Atemluftstroms 17 an mindestens einer Feuchtigkeitserfassungsstelle innerhalb eines dem Atemluftausgang 7 abgewandten Endabschnitts 29 der Atemluftleitung 19 zu erfassen. In ähnlicher Weise kann der Temperatursensor 25 ausgebildet sein, um die Temperatur des Atemluftstroms 17 an mindestens einer Temperaturerfassungsstelle innerhalb des gleichen Endabschnitts 29 zu erfassen. Dabei kann sich der Endabschnitt 29 - in Längsrichtung der Atemluftleitung 19 betrachtet - ausgehend von dem dem Atemluftausgang 7 abgewandten Ende der Atemluftleitung 19 hin zu einem dem Atemluftausgang 7 zugewandten Ende der Atemluftleitung 19 über höchstens ein Drittel, höchstens ein Viertel oder höchstens ein Zehntel einer Länge der Atemluftleitung 19 erstrecken. Dies ermöglicht es, die beiden Sensoren 23, 25 möglichst nah am Patienten zu platzieren.

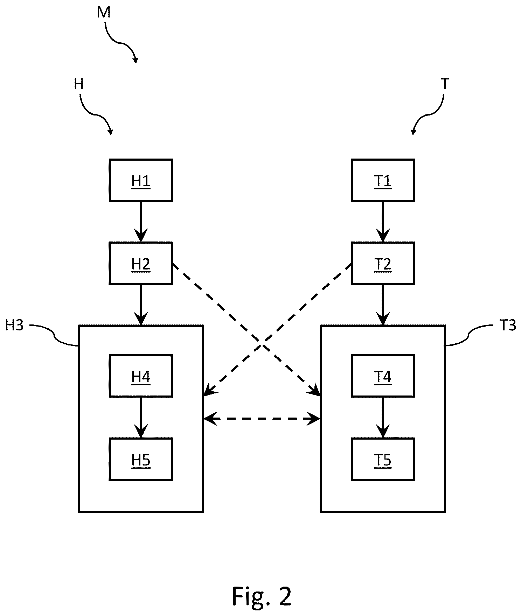

Die Steuereinheit 27 ist konfiguriert, um ein Verfahren M (siehe Fig. 2) zum Steuern der Feuchtigkeit und der Temperatur des Atemluftstroms 17 auszuführen. Dazu kann die Steuereinheit 27 beispielsweise einen Prozessor 31 und einen Speicher 33 umfassen, in dem ein entsprechendes Computerprogramm gespeichert sein kann. Der Prozessor 31 kann konfiguriert sein, um das Computerprogramm auszuführen, wodurch das Verfahren M ausgeführt wird.

Das Verfahren M umfasst zum einen ein Steuern der Feuchtigkeit des Atemluftstroms 17 in mehreren aufeinanderfolgenden Zeitschritten in einem Feuchtigkeitsregelkreis H und zum anderen ein Steuern der Temperatur des Atemluftstroms 17 in mehreren aufeinanderfolgenden Zeitschritten in einem Temperaturregelkreis T, beispielsweise parallel zum Steuern der Feuchtigkeit.

Jeder Zeitschritt des Feuchtigkeitsregelkreises H kann die folgenden Verfahrensschritte umfassen.

In einem Schritt H1 werden ein Feuchtigkeitssollwert 35 und ein Feuchtigkeitsistwert 37, der eine mittels des Feuchtigkeitssensors 23 erfasste Feuchtigkeit des Atemluftstroms 17 anzeigt, empfangen. Der Feuchtigkeitssollwert 35 kann beispielsweise eine relative Luftfeuchtigkeit von 100 % oder weniger, von 98 % oder weniger oder von 95 % oder weniger anzeigen.

In einem Schritt H2 wird eine Feuchtigkeitsabweichung durch Vergleichen des Feuchtigkeitssollwerts 35 mit dem Feuchtigkeitsistwert 37 bestimmt.

In einem Schritt H3 wird mindestens ein Feuchtigkeitssteuerparameter 39 zum Steuern einer Leistung, mit der die Befeuchtungseinrichtung 11 die Flüssigkeit 13 in den Wasserdampf 15 umwandelt, angepasst, um die Feuchtigkeitsabweichung zu verringern.

In entsprechender Weise kann jeder Zeitschritt des Temperaturregelkreises T die folgenden Verfahrensschritte umfassen.

In einem Schritt T1 werden ein Temperatursollwert 41 und ein Temperaturistwert 43, der eine mittels des Temperatursensors 25 erfasste Temperatur des Atemluftstroms 17 anzeigt, empfangen. Der Temperatursollwert 41 kann beispielsweise eine Temperatur zwischen 30 °C und 40 °C anzeigen.

In einem Schritt T2 wird eine Temperaturabweichung durch Vergleichen des Temperatursollwerts 41 mit dem Temperaturistwert 43 bestimmt.

In einem Schritt T3 wird mindestens ein Temperatursteuerparameter 45 zum Steuern einer Leistung, mit der die Temperiereinrichtung 21 den Atemluftstrom 17 zusätzlich temperiert, angepasst, um die Temperaturabweichung zu verringern.

Es ist möglich, dass im Schritt H3 der mindestens eine Feuchtigkeitssteuerparameter 39 unter zusätzlicher Berücksichtigung der Temperaturabweichung und/oder des mindestens einen Temperatursteuerparameters 45 (genauer einer Einstellung des jeweiligen Temperatursteuerparameters 45) aus mindestens einem der Zeitschritte des Temperaturregelkreises T angepasst wird.

Dabei kann der jeweilige Feuchtigkeitssteuerparameter 39 unter zusätzlicher Berücksichtigung der Temperaturabweichung allein oder unter zusätzlicher Berücksichtigung sowohl der Temperaturabweichung als auch des jeweiligen Temperatursteuerparameters 45 derart angepasst werden, dass nicht nur die Feuchtigkeitsabweichung, sondern (in einem begrenzten Umfang) auch die Temperaturabweichung verringert wird.

Beispielsweise wird der jeweilige Feuchtigkeitssteuerparameter 39 nur dann angepasst, um auf diese Weise zusätzlich die Temperaturabweichung zu verringern, wenn im jeweiligen Zeitschritt des Feuchtigkeitsregelkreises H erkannt wird, dass die Temperiereinrichtung 21 bereits deaktiviert ist und/oder die Temperaturabweichung einen bestimmten Schwellenwert erreicht hat.

Zusätzlich oder alternativ ist es möglich, dass der mindestens eine Temperatursteuerparameter 45 unter zusätzlicher Berücksichtigung der Feuchtigkeitsabweichung und/oder des mindestens einen Feuchtigkeitssteuerparameters 39 (genauer einer Einstellung des jeweiligen Feuchtigkeitssteuerparameters 39) aus mindestens einem der Zeitschritte des Feuchtigkeitsregelkreises H angepasst wird.

Dabei kann der jeweilige Temperatursteuerparameter 45 unter zusätzlicher Berücksichtigung der Feuchtigkeitsabweichung allein oder unter zusätzlicher Berücksichtigung sowohl der Feuchtigkeitsabweichung als auch des jeweiligen Feuchtigkeitssteuerparameters 39 derart angepasst werden, dass nicht nur die Temperaturabweichung, sondern (in einem begrenzten Umfang) auch die Feuchtigkeitsabweichung verringert wird.

Beispielsweise wird der jeweilige Temperatursteuerparameter 45 nur dann angepasst, um auf diese Weise zusätzlich die Feuchtigkeitsabweichung zu verringern, wenn im jeweiligen Zeitschritt des Temperaturregelkreises T erkannt wird, dass die Befeuchtungseinrichtung 11 bereits deaktiviert ist und/oder die Feuchtigkeitsabweichung einen bestimmten Schwellenwert erreicht hat.

Zudem ist es möglich, dass in jedem Zeitschritt des Feuchtigkeitsregelkreises H im Schritt H1 und/oder in jedem Zeitschritt des Temperaturregelkreises T im Schritt T1 zusätzlich ein Gewichtungswert 47, der eine Gewichtung des Feuchtigkeitsregelkreises H gegenüber dem Temperaturregelkreis T (beispielsweise eine Gewichtung zwischen 0 % und 100 %) anzeigt, empfangen wird. In diesem Fall kann das Anpassen des jeweiligen Steuerparameters 39 bzw. 45 im jeweiligen Zeitschritt unter zusätzlicher Berücksichtigung des Gewichtungswerts 47 erfolgen.

Beispielsweise kann das Anpassen des jeweiligen Feuchtigkeitssteuerparameters 39 unter zusätzlicher Berücksichtigung des Gewichtungswerts 47 im Schritt H3 einen Schritt H4 und einen Schritt H5 umfassen. Dabei kann im Schritt H4 ein erster Wertebereich mit möglichen Werten für den jeweiligen Feuchtigkeitssteuerparameter 39 unter Verwendung des Gewichtungswerts 47 und einer ersten Zuordnungsvorschrift, die möglichen Gewichtungswerten 47 jeweils einen von mehreren möglichen ersten Wertebereichen zuordnet, bestimmt werden. Hierauf kann im Schritt H5 das Anpassen des jeweiligen Feuchtigkeitssteuerparameters 39 unter Verwendung des ersten Wertebereichs erfolgen.

In entsprechender Weise kann das Anpassen des jeweiligen Temperatursteuerparameters 45 unter zusätzlicher Berücksichtigung des Gewichtungswerts 47 im Schritt T3 einen Schritt T4 und einen Schritt T5 umfassen. Dabei kann im Schritt T4 ein zweiter Wertebereich mit möglichen Werten für den jeweiligen Temperatursteuerparameter 45 unter Verwendung des Gewichtungswerts 47 und einer zweiten Zuordnungsvorschrift, die möglichen Gewichtungswerten 47 jeweils einen von mehreren möglichen zweiten Wertebereichen zuordnet, bestimmt werden. Hierauf kann im Schritt T5 das Anpassen des jeweiligen Temperatursteuerparameters 45 unter Verwendung des zweiten Wertebereichs erfolgen.

Die Kopplung der beiden Regelkreise H, T kann beispielsweise beinhalten, dass die Leistung der Befeuchtungseinrichtung 11, beispielsweise deren Heizleistung, durch Anpassen des mindestens einen Feuchtigkeitssteuerparameters 39 in geeigneter Weise verringert wird oder die Befeuchtungseinrichtung 11 deaktiviert wird, wenn der Temperaturistwert 43 den Temperatursollwert 41 überschreitet und die Leistung der Temperiereinrichtung 21, beispielsweise deren Heizleistung, bereits null ist (beispielsweise weil die Temperiereinrichtung 21 bereits deaktiviert wurde).

Umgekehrt ist es möglich, dass die Leistung der Temperiereinrichtung 21, beispielsweise deren Heizleistung, durch Anpassen des mindestens einen Temperatursteuerparameters 45 in geeigneter Weise verringert wird oder die Temperiereinrichtung 21 deaktiviert wird, wenn der Feuchtigkeitsistwert 37 den Feuchtigkeitssollwert 35 unterschreitet (je nach Gewichtung der beiden Regelkreise H, T). Die Atemluftleitung 19 kann dabei beispielsweise auch als passive Kühleinrichtung fungieren. Dazu kann die Atemluftleitung 19 zusätzlich mit einem entsprechenden Kühlkörper, beispielsweise aus Metall, ausgebildet sein.

Abschließend wird darauf hingewiesen, dass Begriffe wie "aufweisen", "umfassen", "einschließen", "mit" usw. keine anderen Elemente oder Schritte ausschließen und unbestimmte Artikel wie "ein" oder "eine" keine Vielzahl ausschließen.

Ferner wird darauf hingewiesen, dass Merkmale oder Schritte, die mit Verweis auf eine der vorstehenden Ausführungsformen beschrieben sind, auch in Kombination mit Merkmalen oder Schritten, die mit Verweis auf andere der vorstehenden Ausführungsformen beschrieben sind, verwendet werden können.

Bezugszeichen in den Ansprüchen sind nicht als Beschränkung des Umfangs des durch die Ansprüche definierten Gegenstands zu verstehen.

### Liste der Bezugszeichen

- 1: Atemluftbefeuchtungssystem
- 3: Atemluftbefeuchter
- 5: Atemlufteingang
- 7: Atemluftausgang
- 9: Flüssigkeitsbehälter
- 11: Befeuchtungseinrichtung
- 13: Flüssigkeit
- 15: Wasserdampf
- 17: Atemluftstrom
- 19: Atemluftleitung
- 21: Temperiereinrichtung
- 23: Feuchtigkeitssensor
- 25: Temperatursensor
- 27: Steuereinheit
- 29: Endabschnitt
- 31: Prozessor
- 33: Speicher
- 35: Feuchtigkeitssollwert
- 37: Feuchtigkeitsistwert
- 39: Feuchtigkeitssteuerparameter
- 41: Temperatursollwert
- 43: Temperaturistwert
- 45: Temperatursteuerparameter
- 47: Gewichtungswert
- H: Feuchtigkeitsregelkreis
- H1-5: Schritte des Feuchtigkeitsregelkreises
- T: Temperaturregelkreis
- T1-5: Schritte des Temperaturregelkreises

## Patentansprüche

1. Verfahren (M) zum Steuern einer Feuchtigkeit und einer Temperatur eines Atemluftstroms (17) mittels einer Steuereinheit (27) eines Atemluftbefeuchtungssystems (1), wobei das Atemluftbefeuchtungssystem (1) zusätzlich zur Steuereinheit (27) umfasst:
einen Atemluftbefeuchter (3) mit einem Atemlufteingang (5), einem Atemluftausgang (7), einem Flüssigkeitsbehälter (9) und einer elektrisch steuerbaren Befeuchtungseinrichtung (11), die ausgebildet ist, um eine wasserhaltige Flüssigkeit (13) aus dem Flüssigkeitsbehälter (9) in Wasserdampf (15) und/oder Wassertröpfchen umzuwandeln, wobei der Atemluftbefeuchter (3) ausgebildet ist, um Atemluft, die am Atemlufteingang (5) zugeführt wird, durch Vermischen mit dem Wasserdampf (15) und/oder den Wassertröpfchen zu befeuchten und im derart befeuchteten Zustand als den Atemluftstrom (17) am Atemluftausgang (7) bereitzustellen;
eine elektrisch steuerbare Temperiereinrichtung (21), die ausgebildet ist, um den Atemluftstrom (17) zusätzlich zu temperieren;
einen Feuchtigkeitssensor (23) zum Erfassen der Feuchtigkeit des Atemluftstroms (17);
einen Temperatursensor (25) zum Erfassen der Temperatur des Atemluftstroms (17);
wobei das Verfahren (M) umfasst:
Steuern (H1, H2, H3, H4, H5) der Feuchtigkeit des Atemluftstroms (17) in mehreren aufeinanderfolgenden Zeitschritten in einem Feuchtigkeitsregelkreis (H), wobei in jedem Zeitschritt des Feuchtigkeitsregelkreises (H) ein Feuchtigkeitssollwert (35) und ein Feuchtigkeitsistwert (37), der eine mittels des Feuchtigkeitssensors (23) erfasste Feuchtigkeit des Atemluftstroms (17) anzeigt, empfangen werden, eine Feuchtigkeitsabweichung durch Vergleichen des Feuchtigkeitssollwerts (35) mit dem Feuchtigkeitsistwert (37) bestimmt wird und mindestens ein Feuchtigkeitssteuerparameter (39) zum Steuern einer Leistung, mit der die Befeuchtungseinrichtung (11) die Flüssigkeit (13) in den Wasserdampf (15) und/oder die Wassertröpfchen umwandelt, angepasst wird, um die Feuchtigkeitsabweichung zu verringern;
Steuern (T1, T2, T3, T4, T5) der Temperatur des Atemluftstroms (17) in mehreren aufeinanderfolgenden Zeitschritten in einem Temperaturregelkreis (T), wobei in jedem Zeitschritt des Temperaturregelkreises (T) ein Temperatursollwert (41) und ein Temperaturistwert (43), der eine mittels des Temperatursensors (25) erfasste Temperatur des Atemluftstroms (17) anzeigt, empfangen werden, eine Temperaturabweichung durch Vergleichen des Temperatursollwerts (41) mit dem Temperaturistwert (43) bestimmt wird und mindestens ein Temperatursteuerparameter (45) zum Steuern einer Leistung, mit der die Temperiereinrichtung (21) den Atemluftstrom (17) zusätzlich temperiert, angepasst wird, um die Temperaturabweichung zu verringern;
wobei der mindestens eine Feuchtigkeitssteuerparameter (39) unter zusätzlicher Berücksichtigung der Temperaturabweichung und/oder des mindestens einen Temperatursteuerparameters (45) aus mindestens einem der Zeitschritte des Temperaturregelkreises (T) angepasst wird und/oder
wobei der mindestens eine Temperatursteuerparameter (45) unter zusätzlicher Berücksichtigung der Feuchtigkeitsabweichung und/oder des mindestens einen Feuchtigkeitssteuerparameters (39) aus mindestens einem der Zeitschritte des Feuchtigkeitsregelkreises (H) angepasst wird.

2. Verfahren (M) nach Anspruch 1,
wobei der mindestens eine Feuchtigkeitssteuerparameter (39) unter zusätzlicher Berücksichtigung der Temperaturabweichung aus mindestens einem der Zeitschritte des Temperaturregelkreises (T) oder unter zusätzlicher Berücksichtigung sowohl der Temperaturabweichung als auch des mindestens einen Temperatursteuerparameters (45) aus mindestens einem der Zeitschritte des Temperaturregelkreises (T) angepasst wird, um zusätzlich die Temperaturabweichung zu verringern, und/oder
wobei der mindestens eine Temperatursteuerparameter (45) unter zusätzlicher Berücksichtigung der Feuchtigkeitsabweichung aus mindestens einem der Zeitschritte des Feuchtigkeitsregelkreises (H) oder unter zusätzlicher Berücksichtigung sowohl der Feuchtigkeitsabweichung als auch des mindestens einen Feuchtigkeitssteuerparameters (39) aus mindestens einem der Zeitschritte des Feuchtigkeitsregelkreises (H) angepasst wird, um zusätzlich die Feuchtigkeitsabweichung zu verringern.

3. Verfahren (M) nach Anspruch 2,
wobei der mindestens eine Feuchtigkeitssteuerparameter (39) nur dann angepasst wird, um zusätzlich die Temperaturabweichung zu verringern, wenn im jeweiligen Zeitschritt des Feuchtigkeitsregelkreises (H) erkannt wird, dass die Temperiereinrichtung (21) deaktiviert ist und/oder die Temperaturabweichung einen bestimmten Schwellenwert erreicht, und/oder
wobei der mindestens eine Temperatursteuerparameter (45) nur dann angepasst wird, um zusätzlich die Feuchtigkeitsabweichung zu verringern, wenn im jeweiligen Zeitschritt des Temperaturregelkreises (T) erkannt wird, dass die Befeuchtungseinrichtung (11) deaktiviert ist und/oder die Feuchtigkeitsabweichung einen bestimmten Schwellenwert erreicht.

4. Verfahren (M) nach einem der vorhergehenden Ansprüche,
wobei in jedem Zeitschritt des Feuchtigkeitsregelkreises (H) und/oder des Temperaturregelkreises (T) zusätzlich ein Gewichtungswert (47), der eine Gewichtung des Feuchtigkeitsregelkreises (H) gegenüber dem Temperaturregelkreis (T) anzeigt, empfangen wird und das Anpassen (H3, T3) des mindestens einen Feuchtigkeitssteuerparameters (39) und/oder des mindestens einen Temperatursteuerparameters (45) im jeweiligen Zeitschritt unter zusätzlicher Berücksichtigung des Gewichtungswerts (47) erfolgt.

5. Verfahren (M) nach Anspruch 4,
wobei das Anpassen (H3) des mindestens einen Feuchtigkeitssteuerparameters (39) unter zusätzlicher Berücksichtigung des Gewichtungswerts (47) umfasst:
Bestimmen (H4) eines ersten Wertebereichs mit möglichen Werten für den mindestens einen Feuchtigkeitssteuerparameter (39) unter Verwendung des Gewichtungswerts (47) und einer ersten Zuordnungsvorschrift, die möglichen Gewichtungswerten (47) jeweils einen von mehreren möglichen ersten Wertebereichen zuordnet;
Anpassen (H5) des mindestens einen Feuchtigkeitssteuerparameters (39) unter Verwendung des ersten Wertebereichs.

6. Verfahren (M) nach Anspruch 4 oder 5,
wobei das Anpassen (T3) des mindestens einen Temperatursteuerparameters (45) unter zusätzlicher Berücksichtigung des Gewichtungswerts (47) umfasst:
Bestimmen (T4) eines zweiten Wertebereichs mit möglichen Werten für den mindestens einen Temperatursteuerparameter (45) unter Verwendung des Gewichtungswerts (47) und einer zweiten Zuordnungsvorschrift, die möglichen Gewichtungswerten (47) jeweils einen von mehreren möglichen zweiten Wertebereichen zuordnet;
Anpassen (T5) des mindestens einen Temperatursteuerparameters (45) unter Verwendung des zweiten Wertebereichs.

7. Verfahren (M) nach einem der vorhergehenden Ansprüche,
wobei der Feuchtigkeitssollwert (35) eine relative Luftfeuchtigkeit von 98 % oder weniger oder von 95 % oder weniger anzeigt.

8. Verfahren (M) nach einem der vorhergehenden Ansprüche,
wobei der Temperatursollwert (41) eine Temperatur zwischen 30 °C und 40 °C, vorzugsweise zwischen 37 °C und 39 °C, anzeigt.

9. Steuereinheit (27) für ein Atemluftbefeuchtungssystem (1), wobei das Atemluftbefeuchtungssystem (1) umfasst:
einen Atemluftbefeuchter (3) mit einem Atemlufteingang (5), einem Atemluftausgang (7), einem Flüssigkeitsbehälter (9) und einer elektrisch steuerbaren Befeuchtungseinrichtung (11), die ausgebildet ist, um eine wasserhaltige Flüssigkeit (13) aus dem Flüssigkeitsbehälter (9) in Wasserdampf (15) und/oder Wassertröpfchen umzuwandeln, wobei der Atemluftbefeuchter (3) ausgebildet ist, um Atemluft, die am Atemlufteingang (5) zugeführt wird, durch Vermischen mit dem Wasserdampf (15) und/oder den Wassertröpfchen zu befeuchten und im derart befeuchteten Zustand als einen Atemluftstrom (17) am Atemluftausgang (7) bereitzustellen;
eine elektrisch steuerbare Temperiereinrichtung (21), die ausgebildet ist, um den Atemluftstrom (17) zusätzlich zu temperieren;
einen Feuchtigkeitssensor (23) zum Erfassen einer Feuchtigkeit des Atemluftstroms (17);
einen Temperatursensor (25) zum Erfassen einer Temperatur des Atemluftstroms (17);
wobei die Steuereinheit (27) konfiguriert ist, um das Verfahren (M) nach einem der vorhergehenden Ansprüche auszuführen.

10. Atemluftbefeuchtungssystem (1), umfassend:
einen Atemluftbefeuchter (3) mit einem Atemlufteingang (5), einem Atemluftausgang (7), einem Flüssigkeitsbehälter (9) und einer elektrisch steuerbaren Befeuchtungseinrichtung (11), die ausgebildet ist, um eine wasserhaltige Flüssigkeit (13) aus dem Flüssigkeitsbehälter (9) in Wasserdampf (15) und/oder Wassertröpfchen umzuwandeln, wobei der Atemluftbefeuchter (3) ausgebildet ist, um Atemluft, die am Atemlufteingang (5) zugeführt wird, durch Vermischen mit dem Wasserdampf (15) und/oder den Wassertröpfchen zu befeuchten und im derart befeuchteten Zustand als einen Atemluftstrom (17) am Atemluftausgang (7) bereitzustellen;
eine Atemluftleitung (19) zum Anschließen an den Atemluftausgang (7), sodass der Atemluftstrom (17) durch die Atemluftleitung (19) geleitet wird;
eine elektrisch steuerbare Temperiereinrichtung (21), die ausgebildet ist, um den Atemluftstrom (17) zusätzlich zu temperieren;
einen Feuchtigkeitssensor (23) zum Erfassen einer Feuchtigkeit des Atemluftstroms (17);
einen Temperatursensor (25) zum Erfassen einer Temperatur des Atemluftstroms (17);
eine Steuereinheit (27) nach Anspruch 9.

11. Atemluftbefeuchtungssystem (1) nach Anspruch 10,
wobei die Temperiereinrichtung (21) ausgebildet ist, um den Atemluftstrom (17) durch Erwärmen und/oder Kühlen zumindest eines Abschnitts der Atemluftleitung (19) zusätzlich zu temperieren.

12. Atemluftbefeuchtungssystem (1) nach Anspruch 10 oder 11,
wobei der Feuchtigkeitssensor (23) ausgebildet ist, um die Feuchtigkeit des Atemluftstroms (17) an mindestens einer Feuchtigkeitserfassungsstelle innerhalb eines dem Atemluftausgang (7) abgewandten Endabschnitts (29) der Atemluftleitung (19) zu erfassen, und/oder wobei der Temperatursensor (25) ausgebildet ist, um die Temperatur des Atemluftstroms (17) an mindestens einer Temperaturerfassungsstelle innerhalb eines dem Atemluftausgang (7) abgewandten Endabschnitts (29) der Atemluftleitung (19) zu erfassen;
wobei der Endabschnitt (29) ein Längsabschnitt der Atemluftleitung (19) ist, der sich ausgehend von einem dem Atemluftausgang (7) abgewandten Ende der Atemluftleitung (19) hin zu einem dem Atemluftausgang (7) zugewandten Ende der Atemluftleitung (19) über höchstens ein Drittel, höchstens ein Viertel oder höchstens ein Zehntel einer Länge der Atemluftleitung (19) erstreckt.

13. Computerprogramm, umfassend Befehle, die die Steuereinheit (27) nach Anspruch 9 beim Ausführen des Computerprogramms durch die Steuereinheit (27) veranlassen, das Verfahren (M) nach einem der Ansprüche 1 bis 8 auszuführen.

14. Computerlesbares Medium, auf dem das Computerprogramm nach Anspruch 13 gespeichert ist.
